# EUROPEAN PATENT SPECIFICATION

(11) **EP 1 627 605 B1**
(45) Date of publication and mention of the grant of the patent: **01.12.2010**
(21) Application number: 05254699.1
(22) Date of filing: 27.07.2005
(51) Int. Cl.: A61B 17/072, A61M 1/00

(54) **Surgical stapling instrument having a medicament dispenser**
Mit Artzneimittelspender versehene, chirurgische Klammersetzvorrichtung
Agrafeuse chirurgicale avec distributeur de médicaments

(30) Priority: 28.07.2004 US 591694 P; 17.03.2005 US 82495; 21.06.2005 US 157767
(43) Date of publication of application: 22.02.2006
(73) Proprietor: ETHICON ENDO-SURGERY, INC., Cincinnati, Ohio 45242 (US)
(72) Inventor: Shelton IV, Frederick E., Hillsboro, Ohio 45133 (US); Hueil, Joseph C., Loveland, Ohio 45140 (US); Morgan, Jerome R., Cincinnati, Ohio 45236 (US)
(74) Representative: Tunstall, Christopher Stephen

(56) References cited:
- EP-A- 0 741 996
- WO-A-01/62158
- WO-A-03/094743
- US-A1- 2003 065 358

## Description

### FIELD OF THE INVENTION

The present invention relates in general to surgical stapler instruments that are capable of applying lines of staples to tissue while cutting the tissue between those staple lines and, more particularly, to improvements relating to stapler instruments and improvements in processes for forming various components of such stapler instruments.

### BACKGROUND OF THE INVENTION

Surgical staplers have been used in the prior art to simultaneously make a longitudinal incision in tissue and apply lines of staples on opposing sides of the incision. Such instruments commonly include a pair of cooperating jaw members that, if the instrument is intended for endoscopic or laparoscopic applications, are capable of passing through a cannula passageway. One of the jaw members receives a staple cartridge having at least two laterally spaced rows of staples. The other jaw member defines an anvil having staple-forming pockets aligned with the rows of staples in the cartridge. The instrument includes a plurality of reciprocating wedges which, when driven distally, pass through openings in the staple cartridge and engage drivers supporting the staples to effect the firing of the staples toward the anvil.

An example of a surgical stapler suitable for endoscopic applications, described in U.S. Pat. No. 5,465,895, advantageously provides distinct closing and firing actions.

Thereby, a clinician is able to close the jaw members upon tissue to position the tissue prior to firing. Once the clinician has determined that the jaw members are properly gripping tissue, the clinician can then fire the surgical stapler, thereby severing and stapling the tissue. The simultaneous severing and stapling avoids complications that may arise when performing such actions sequentially with different surgical tools that respectively only sever or staple.

These minimally invasive surgical instruments have been widely used and have proven to be a significant advancement over traditional open surgical techniques. It would be desirable to incorporate yet additional features and capabilities. For instance, in Int'1 Pat. Appln. WO 03/094743 A1, a wound closure material applicator assembly is described that dispenses at a needle in a knife of a surgical stapling apparatus as the knife is moved to sever tissue. Dispensing is actuated by reservoir compressed by the firing handle or by a separate syringe. WO-A-0162158 is considered to represent the closest prior art and discloses a surgical cutter/stapler with in combination the following features of appended claim 1: a handle, an elongate shaft, a firing bar, a cutting edge, a staple cartridge, electrical control circuitry, a fluid dispenser (directly associated with the cutting/firing mechanism) comprising an internal volume adapted to be filled with a medical substance, a dispensing opening and a plunger configured to extend into the internal volume to dispense the medical substance through the dispensing opening when electrically activated (along with advancement of the cutting edge and firing of the staples). US 2003/0065358 A1 discloses a surgical apparatus having an electroactive polymer actuator for actuating an end effector of the apparatus.

While such an ability to apply a medical substance upon tissue simultaneously with severing and stapling may be desirable, it is believed that it would be desirable to not impose a greater force to fire requirement upon the surgeon, or to require another detached device be actuated.

Consequently, a significant need exists for a surgical instrument with an improved ability to dispense a medical substance.

### BRIEF SUMMARY OF THE INVENTION

The invention overcomes the above-noted and other deficiencies of the prior art by providing a surgical instrument that is suitable for minimally invasive surgical procedures by having a handle that positions an end effector through a surgical opening via an elongate shaft. An electrically controlled medical substance delivery mechanism incorporated through the elongate shaft to the end effector enhances the utility and effectiveness of the instrument. Thereby, numerous therapeutic treatments may be precisely applied onto the tissue as it is severed so that post operative recovery and complications are reduced. The present invention provides a surgical stapling and severing instrument as defined in appended claim 1. Preferred embodiments are defined in appended claims 2 to 8.

This end effector includes opposing jaws for clamping tissue. A firing bar is received for reciprocating longitudinal motion in the elongate shaft to transfer a firing motion from the handle. A cutting surface distally attached to the firing bar is pushed by this firing motion to sever the clamped tissue in the end effector. Enhancing healing of the severed tissue, a fluid passage is advantageously defined longitudinally in the firing bar to the cutting surface. An electrical fluid dispenser in communication with the fluid passage responds to a dispensing signal from control circuitry to dispense a medical substance along the fluid passage to the cutting surface.

The surgical instrument includes a fluid passage to the end effector. A syringe cylinder in the surgical instrument has a portion of its internal volume filled with a medical substance. Control circuitry generates a dispensing signal to an electroactive polymer plunger in the syringe cylinder. The electroactive polymer plunger expands into the internal volume to dispense the medical substance through a dispensing opening through the fluid passage to the end effector. Thereby, a number of minimally invasive surgical procedures may be accompanied by simultaneous or selective dispensing of a medical substance at the site of the surgical treatment. Moreover, the characteristics of electroactive polymers lends themselves to a reliable instrument with significant shelf life prior to use.

The surgical instrument that staples and severs clamped tissue benefits from the simultaneous electrically actuated dispensing of a medical substance at a cutting surface of a firing bar. Thereby, trauma to the tissue due to the severing and stapling may be mitigated by a capability of dispensing directly onto this location in a timely fashion.

These and other objects and advantages of the present invention shall be made apparent from the accompanying drawings and the description thereof.

### BRIEF DESCRIPTION OF THE FIGURES

The accompanying drawings, which are incorporated in and constitute a part of this specification, illustrate embodiments of the invention, and, together with the general description of the invention given above, and the detailed description of the embodiments given below, serve to explain the principles of the present invention.

FIG. 1 is a perspective view of a surgical stapling and severing instrument having a fluid actuated upper jaw (anvil) in an open position and an electroactive polymer (EAP) medical substance dispensing shaft.

FIG. 2 is a disassembled perspective view of an implement portion of the surgical stapling and severing instrument of FIG. 1.

FIG. 3 is left side view in a elevation of the implement portion of the surgical stapling and severing instrument of FIG. 1 taken in cross section generally through a longitudinal axis and passing through an offset EAP syringe and receptacle that is in fluid communication with a dispensing groove in an E-beam firing bar.

FIG. 4 is a left side detail view in elevation of a distal portion of the implement portion of the surgical stapling and severing instrument of FIG. 1 taken in cross section generally through the longitudinal axis thereof but showing a laterally offset fluid bladder actuator opening the anvil.

FIG. 5 is a left side detail view of an E-beam firing bar incorporating medical substance ducting.

FIG. 6 is a left side detail view in elevation of the distal portion of the implement portion of the surgical stapling and severing instrument of FIG. 4 taken in cross section generally through the longitudinal axis thereof with the anvil closed.

FIG. 7 is a left side detail view of the E-beam firing bar of FIG. 6.

FIG. 8 is a top detail view of a joined portion of a lower jaw (staple channel) of the end effector and elongate shaft taken in cross section through the lines 8-8 depicting guidance to the E-beam firing bar.

FIG. 9 is a front view of a firing bar guide of the implement portion of the surgical stapling and severing instrument of FIG. 2.

FIG. 10 is a left side view of the firing bar guide of FIG. 9 taken in cross section along lines 9-9.

FIG. 11 is a front view in elevation of the elongate shaft of the surgical stapling and severing instrument of FIG. 3 taken along lines 11-11 taken through a distal end of the EAP medical substance syringe.

FIG. 12 is a left side view of the EAP medical substance syringe of FIG. 11.

FIG. 13 is a left side view of the implement portion of the surgical stapling and severing instrument of FIG. 1 partially cut away to show proximal mountings for the EAP medical substance syringe.

FIG. 14 is a left side detail view of the EAP medical substance syringe and receptacle of the elongate shaft of the surgical stapling and severing instrument of FIG. 13.

FIG. 15 is a top view of the firing bar of the surgical stapling and severing instrument of FIG. 2.

FIG. 16 is a left side view of a laminate firing bar showing an internal fluid path in phantom for the surgical stapling and severing instrument of FIG. 1.

FIG. 17 is a left side detail view of an alternate E-beam showing an internal fluid path in phantom showing an internal fluid path in phantom.

FIG. 18 is a front view in elevation of the laminate firing bar of FIG. 15 taken in cross section along line 18-18 through a proximal open groove of a fluid path.

### DETAILED DESCRIPTION OF THE INVENTION

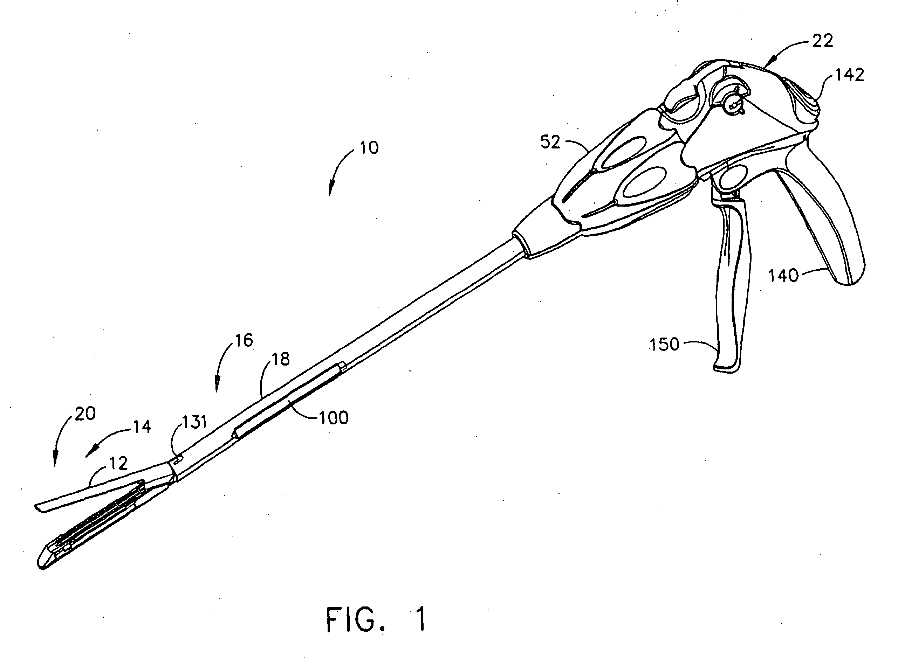

Turning to the Drawings, wherein like numerals denote like components throughout the several views, in FIGS. 1-2, a surgical stapling and severing instrument 10 that is capable of practicing the unique benefits of the present invention, including both fluid actuation (e.g., opening, closing/clamping) of an upper jaw (anvil) 12 of an end effector 14 as well as dispensing a medical substance onto tissue as severed. Fluid actuation of the end effector 14 provides a range of design options that avoid some design limitations of traditional mechanical linkages. For example, instances of binding or component failure may be avoided. Further, dispensing liquids onto severed tissue allows for a range of advantageous therapeutic treatments to be applied, such as the application of anesthetics, adhesives, cauterizing substances, antibiotics, coagulant, etc.

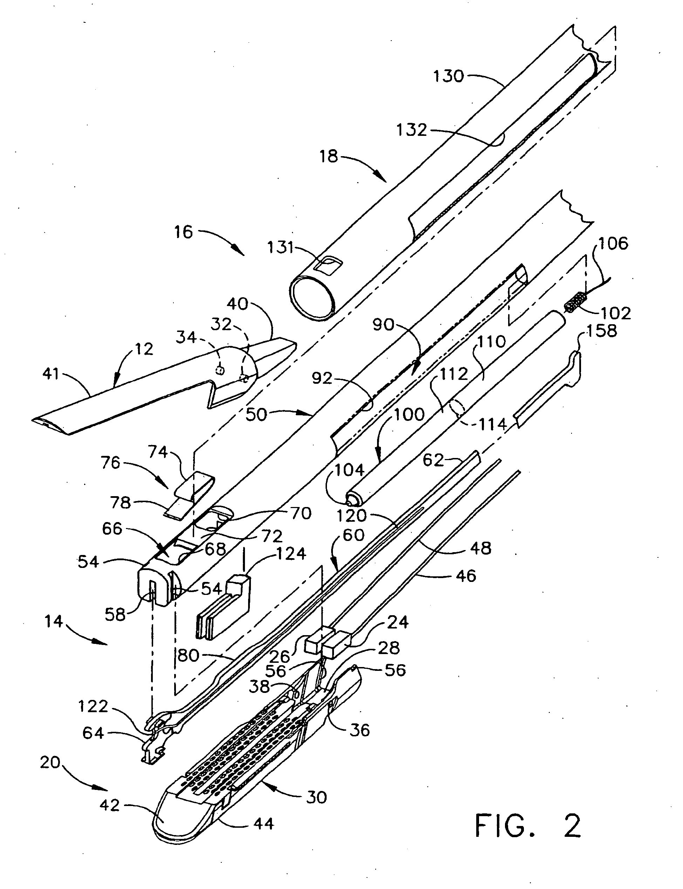

With particular reference to FIG. 2, the surgical stapling and severing instrument 10 includes an implement portion 16 formed by an elongate shaft 18 and the end effector 14, depicted as a stapling assembly 20. The surgical stapling and severing instrument 10 also includes a handle 22 (FIG. 1) attached proximally to the shaft 18. The handle 22 remains external to the patient as the implement portion 16 is inserted through a surgical opening, or especially a cannula of a trocar that forms a pneumoperitoneum for performing a minimally invasive surgical procedure.

Left and right fluid bladders (lift bags) 24, 26 are supported within an aft portion 28 of a staple channel 30. The anvil 12 includes a pair of inwardly directed lateral pivot pins 32, 34 that pivotally engage outwardly open lateral pivot recesses 36, 38 formed in the staple channel 30 distal to the aft portion 28. The anvil 12 includes a proximally directed lever tray 40 that projects into the aft portion 28 of the staple channel 30 overtop and in contact with the fluid bladders (lift bags) 24, 26 such that filling the fluid bladders 24, 26 causes a distal clamping section 41 of the anvil 12 to pivot like a teeter-totter toward a staple cartridge 42 held in a distal portion 44 of the staple channel 30. Evacuation and collapse of the fluid bladders 24, 26, or some other resilient feature of the end effector 14, causes the anvil 12 to open. Left and right fluid conduits 46, 48 communicate respectively with the left and right fluid bladders 24, 26 to bi-directionally transfer fluid for actuation. It should be appreciated that applications consistent with the present invention may include a mechanical actuation in the handle 22 (e.g., closure trigger) (not shown) wherein the user depresses a control that causes closure and clamping of the end effector 12.

It will be appreciated that the terms "proximal" and "distal" are used herein with reference to a clinician gripping a handle of an instrument. Thus, the staple applying assembly 20 is distal with respect to the more proximal handle 22. It will be further appreciated that, for convenience and clarity, spatial terms such as "vertical" and "horizontal" are used herein with respect to the drawings. However, surgical instruments are used in many orientations and positions, and these terms are not intended to be limiting and absolute.

With particular reference to FIG. 2, the elongate shaft 18 includes a frame 50 whose proximal end is rotatably engaged to the handle 22 (FIG. 1) such that a rotation knob 52 rotates the frame 50 along with the end effector 14. A distal end of the frame 50 has lateral recesses 54 that engage a proximal lip 56 of the staple channel 30. The frame 50 includes a laterally centered, bottom firing slot 58 that passes longitudinally through the frame 50 for receiving a two-piece firing bar 60 comprised of a firing bar 62 with a distally attached E-beam 64, the latter translating within the staple applying assembly 20 to sever and staple tissue. A distal portion of the frame 50 includes an upper cavity 66 whose distal and proximal ends communicate through distal and proximal apertures 68, 70, defining there between a cross bar 72 over which a distally projecting clip 74 of a clip spring 76 engages with a lower spring arm 78, distally and downwardly projecting through the upper cavity 66 to bias the firing bar 62 downwardly into engagement with the staple channel 30, especially when the lower spring arm 78 encounters a raised portion 80 on the firing bar 62.

Medical substance dispensing is integrated into the elongate shaft 18 by including a laterally offset cylindrical cavity 90 formed in the frame 50 that communicates along its longitudinal length to the outside via a rectangular aperture 92 that is slightly shorter than an electroactive polymer (EAP) syringe 100 that is inserted through the aperture 92 into the cylindrical cavity 90. A proximal portion of the cylindrical cavity 90 contains a longitudinally aligned compression spring 102 that urges a distal dispensing cone 104 of the EAP syringe 100 distally into sealing contact with the frame 50 and allows translation for insertion and removal of the EAP syringe 100. An electrical conductor 106 passes through the frame 50 and is attached to the compression spring 102, which is also formed of an electrically conductive metal. An aft portion of the EAP syringe 100 is conductive and contacts the spring 102 to form a cathode to an EAP actuator 110 held in a proximal portion of the EAP syringe 100. It will be appreciated that another conductor, perhaps traveling with the conductor 106, also electrically communicates to the EAP actuator 110 to serve as the anode.

When activated, the EAP actuator 110 longitudinally expands, serving as a plunger to dispel a medical substance 112 in a distal portion of the EAP syringe 100 through the distal dispensing cone 104. Insofar as the EAP actuator 110 laterally contracts to compensate for its longitudinal expansion, a plunger seal 114 maintains a transverse seal within the EAP syringe 100. An vent (not shown), such as around conductor 106 allows air to refill the EAP syringe 100 behind the plunger seal 114 as the medical substance 112 is dispensed. The vent may rely upon the surface tension of the medical substance 112 to avoid leaking or be a one-way valve. As described below, the medical substance 112 is conducted by the frame 50 to a lateral fluid groove 120 that is formed in the firing bar 62 and the E-beam 64 to direct the medical substance to a cutting surface 122 of the E-beam 64. The frame slot 58 is sized to seal the lateral fluid groove 120. The portion of the lateral fluid groove 120 that is positioned under the spring clip 76 is sealed by a firing bar guide 124. In the illustrative version, an outer sheath 130 encompasses the frame 50 and proximally projecting lever tray 40 of the anvil 12. A top distal opening 131 allows closing of the anvil 12.

An outer rectangular aperture 132 of the outer sheath 130 is sized and longitudinally positioned to correspond to the rectangular aperture 92 formed in frame 50. In some applications, the outer sheath 130 may be rotated to selectively align the rectangular aperture 92 with the outer rectangular aperture 132 for insertion or removal of the EAP syringe 100. It should be appreciated that in some applications that the EAP syringe 100 may be integrally assembled into an elongate shaft that does not allow for selecting a desired medical substance. For instance, a disposable implement portion with an integral staple cartridge and medical dispensing reservoir may be selected by the clinician as a unit. It is believed that allowing insertion at the time of use, though, has certain advantages including clinical flexibility in selecting a medical substance (e.g., anesthetics, adhesives, antibiotics, cauterizing compound, etc.) and extending the shelf life / simplifying storage and packaging of the implement portion 16.

In the illustrative version, an elongate stack of many disk-shaped EAP layers are aligned longitudinally and configured to expand along this longitudinal axis. Electroactive polymers (EAPs) are a set of conductive doped polymers that change shape when electrical voltage is applied. In essence, the conductive polymer is paired to some form of ionic fluid or gel and electrodes. Flow of the ions from the fluid/gel into or out of the conductive polymer is induced by the voltage potential applied and this flow induces the shape change of the polymer. The voltage potential ranges from 1V to 4kV, depending on the polymer and ionic fluid used. Some of the EAPs contract when voltage is applied and some expand. The EAPs may be paired to mechanical means such as springs or flexible plates to change the effect that is caused when the voltage is applied.

There are two basic types of EAPs and multiple configurations of each type. The two basic types are a fiber bundle and a laminate version. The fiber bundle consists of fibers around 30-50 microns. These fibers may be woven into a bundle much like textiles and are often called EAP yarn because of this. This type of EAP contracts when voltage is applied. The electrodes are usually made up of a central wire core and a conductive outer sheath that also serves to contain the ionic fluid that surrounds the fiber bundles. An example of a commercially available fiber EAP material, manufactured by Santa Fe Science and Technology and sold as PANION™ fiber, is described in U.S. Pat. No. 6,667,825.

The other type is a laminate structure, which consists of a layer of EAP polymer, a layer of ionic gel and two flexible plates that are attached to either side of the laminate. When a voltage is applied, the square laminate plate expands in one direction and contracts in the perpendicular direction. An example of a commercially available laminate (plate) EAP material is from Artificial Muscle Inc, a division of SRI Laboratories. Plate EAP material is manufactured by EAMEX of Japan and is referred to as thin film EAP.

It should be noted that EAPs do not change volume when energized; they merely expand or contract in one direction while doing the opposite in the transverse direction. The laminate version may be used in its basic form by containing one side against a rigid structure and using the other much like a piston. The laminate version may also be adhered to either side of a flexible plate. When one side of the flexible plate EAP is energized, it expands flexing the plate in the opposite direction. This allows the plate to be flexed in either direction, depending on which side is energized.

An EAP actuator usually consists of numerous layers or fibers bundled together to work in cooperation. The mechanical configuration of the EAP determines the EAP actuator and its capabilities for motion. The EAP may be formed into long stands and wrapped around a single central electrode. A flexible exterior outer sleeve will form the other electrode for the actuator as well as contain the ionic fluid necessary for the function of the device. In this configuration when the electrical field is applied to the electrodes, the strands of EAP shorten. This configuration of EAP actuator is called a fiber EAP actuator. Likewise, the laminate configuration may be placed in numerous layers on either side of a flexible plate or merely in layers on itself to increase its capabilities. Typical fiber structures have an effective strain of 2-4% where the typical laminate version achieves 20-30%, utilizing much higher voltages.

For instance, a laminate EAP composite may be formed from a positive plate electrode layer attached to an EAP layer, which in turn is attached to an ionic cell layer, which in turn is attached to a negative plate electrode layer. A plurality of laminate EAP composites may be affixed in a stack by adhesive layers there between to form an EAP plate actuator. It should be appreciated that opposing EAP actuators may be formed that can selectively bend in either direction.

A contracting EAP fiber actuator may include a longitudinal platinum cathode wire that passes through an insulative polymer proximal end cap through an elongate cylindrical cavity formed within a plastic cylinder wall that is conductively doped to serve as a positive anode. A distal end of the platinum cathode wire is embedded into an insulative polymer distal end cap. A plurality of contracting polymer fibers are arranged parallel with and surrounding the cathode wire and have their ends embedded into respective end caps. The plastic cylinder wall is peripherally attached around respective end caps to enclose the cylindrical cavity to seal in ionic fluid or gel that fills the space between contracting polymer fibers and cathode wire. When a voltage is applied across the plastic cylinder wall (anode) and cathode wire, ionic fluid enters the contracting polymer fibers, causing their outer diameter to swell with a corresponding contraction in length, thereby drawing the end caps toward one another.

Returning to FIG. 1, the handle 22 controls closure of the anvil 12, firing of the two-piece firing bar 60 (FIG. 2), and dispensing of the medical substance. In an illustrative version, a pistol grip 140 may be grasped and a thumb button 142 depressed as desired to control closure of the anvil 12. The thumb button 142 provides a proportional electrical signal to an EAP dispensing actuator (not shown) similar to the EAP syringe 100 to transfer fluid through the conduits 46, 48 to the fluid bladders 24, 26 to close the anvil 12 (FIG. 2). When the thumb button 142 is fully depressed, a mechanical toggle lock (not shown) engages to hold the thumb button 142 down until a full depression releases the toggle lock for releasing the thumb button 142. Thus, when the thumb button 142 is held down, the surgeon has a visual indication that the end effector 14 is closed and clamped, which may be maintained in this position by continued activation of an EAP dispensing actuator or by a locking feature. For instance, control circuitry may sense movement of the thumb button 142, causing a normally closed EAP shutoff valve (not shown) to open that communicates between the EAP dispensing actuator and the conduits 46, 48. Once movement ceases, the EAP shutoff valve is allowed to close again, maintaining the anvil 12 position. In addition, a manual release could be incorporated to defeat such a lockout to open the anvil 12.

As an alternative, a closure trigger (not shown) or other actuator may be included that bi-directionally transfers fluid to the fluid bladders 24, 26 as described in commonly owned US 2006190028. A number of such fluid actuators for articulation of a pivoting shaft are described that may be adapted for closing the anvil 12. To take full advantage of the differential fluid transfer described for several of these versions, it should be appreciated that an opposing lift bag (not shown) may be placed above the lever tray 40 of the anvil 12 to assert an opening force as the left and right fluid bladders (lift bags) 24, 26 collapse.

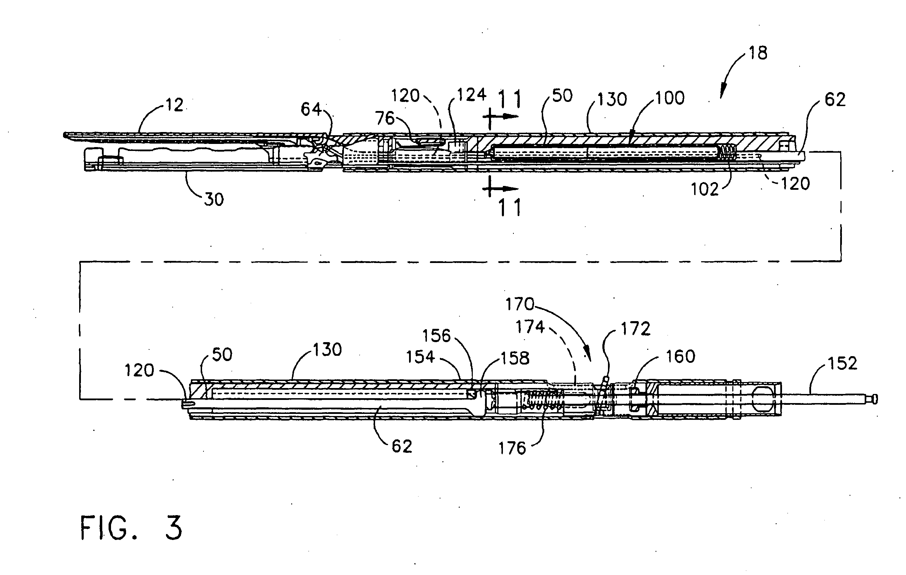

With particular reference to FIG. 3, the handle 22 includes a firing trigger 150 (FIG. 1) that is drawn proximally toward the pistol grip 140 to cause a firing rod 152 to move distally in a proximal portion 154 of the elongate shaft 18. A distal bracket 156 of the firing rod 152 engages an upward proximal hook 158 of the firing bar 62. A dynamic seal 160 within the frame 50 seals to the firing rod 152 so that the implement portion 16 is pneumatically sealed when inserted into an insufflated abdomen.

An anti-backup mechanism 170 of the firing rod 152 may be advantageously included for a handle 22 that includes a multiple stroke firing trigger 150 and a retraction biased firing mechanism coupled to the firing rod 152 (not shown). In particular, an anti-backup locking plate 172 has the firing rod 152 pass through a closely fitting through hole (not shown) that binds when a retracting firing rod 152 tips the lock plate 172 backward as shown with the bottom of the locking plate 172 held in position within the frame 50. An anti-backup cam sleeve 174 is positioned distal to the anti-backup locking plate 172 and urged into contact by a more distal compression spring 176 through which the firing rod 152 passes and that is compressed within the frame 50. It should be appreciated that mechanisms in the handle 22 may manually release the anti-backup mechanism 170 for retraction of the firing rod 152.

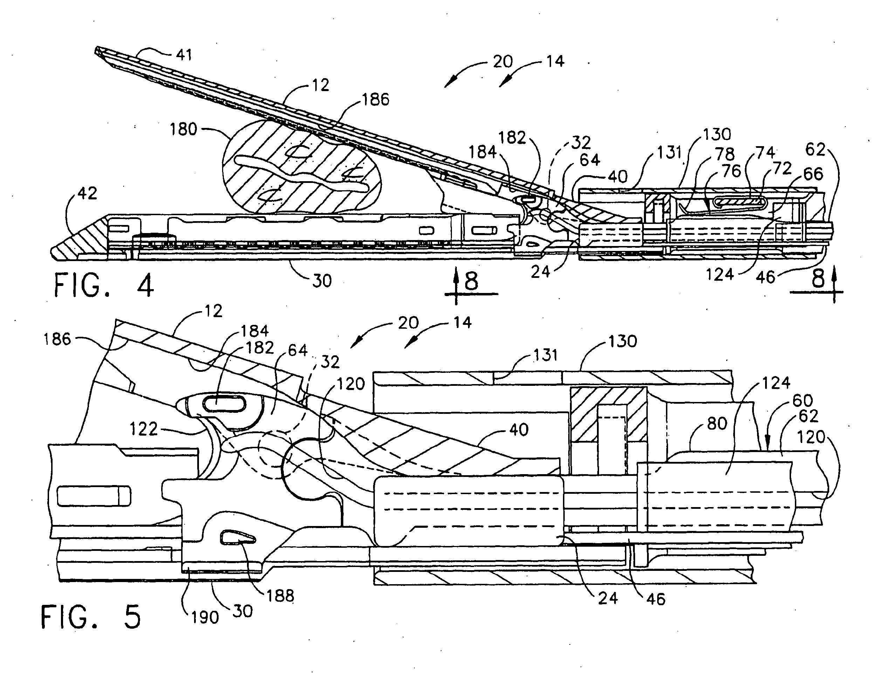

In FIGS. 4-5, the end effector 14, which in the illustrative version is a staple applying assembly 20, is opened by having fluid bladder 24 deflated, drawing down lever tray 40 of the anvil 12, which pivots about pin 32 raising distal clamping section 41 thereby allowing positioning body tissue 180 between the anvil 12 and staple cartridge 42. The E-beam 64 has an upper pin 182 that resides within an anvil pocket 184 allowing repeated opening and closing of the anvil 12. An anvil slot 186 formed along the length of the anvil 12 receives the upper pin 182 when the anvil 12 is closed and the two piece firing bar 60 is distally advanced. A middle pin 188 slides within the staple cartridge 42 above the staple channel 30 in opposition to a bottom pin or foot 190 that slides along a bottom surface of the staple channel 30.

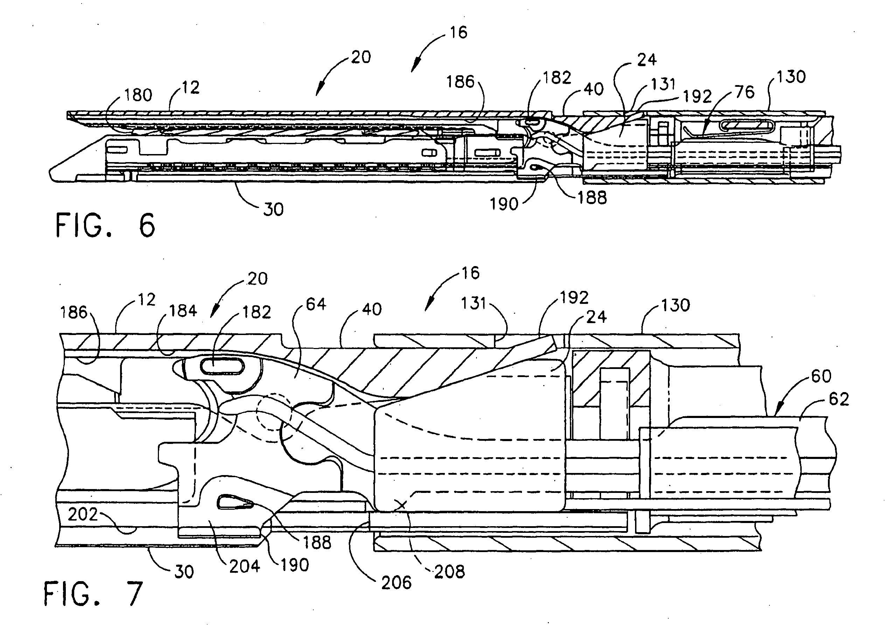

In FIGS. 6-7, the staple applying assembly 20 has been closed by expanding the fluid bladder (lift bag) 24, raising the lever tray 40 of the anvil 12 until flush with the outer sheath 130, with a proximal upwardly bent tip 192 of the lever tray 40 allowed to enter the top distal opening 131. This bent tip 192 in combination with the opening 131, advantageously allows greater radial travel for the anvil 12 as well as presenting an abutting surface rather than a piercing tip to the underlying fluid bladder 24. When the anvil 12 is closed, the upper pin 182 is aligned with the anvil slot 186 for firing and the tissue 180 is flattened to a thickness appropriate for severing and stapling.

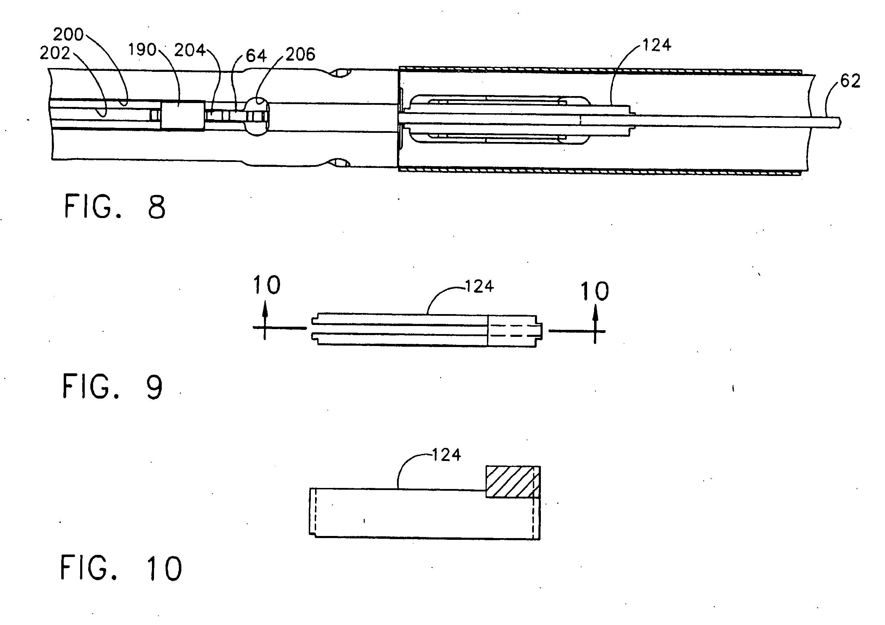

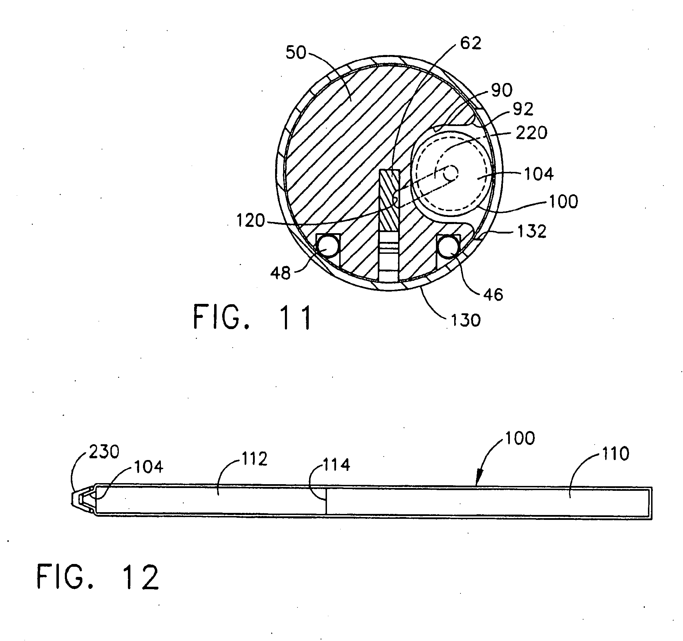

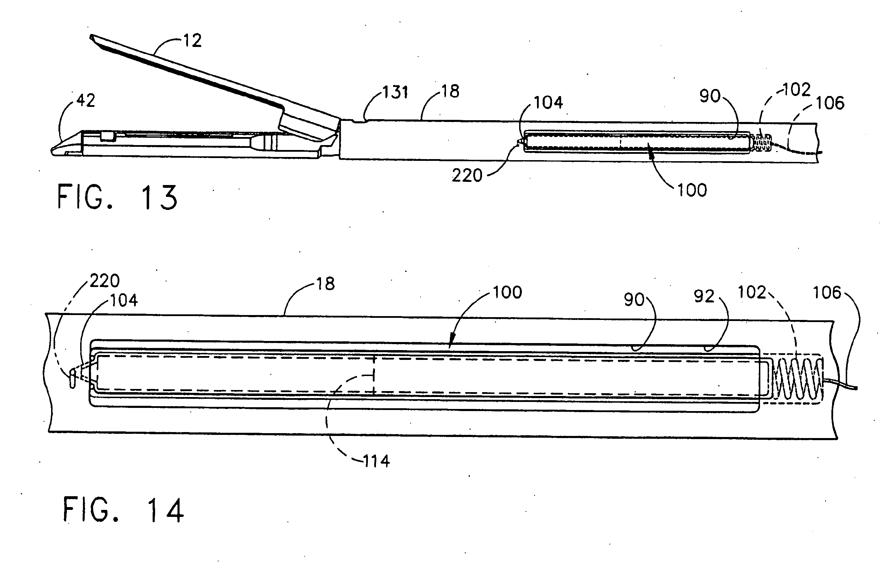

In FIGS. 7-8, the E-beam 64 is cut away to show its bottom foot 190 riding along a downwardly open laterally widened recess 200 that communicates with a narrow longitudinal slot 202 through which a vertical portion 204 of the E-beam 64 passes. A proximal aperture 206 to the narrow longitudinal slot 202 allows an assembly entrance for the lower foot 190. A bottom bump 208 is positioned on the firing bar 62 to drop into the proximal aperture 206 during an initial portion of firing travel under the urging of the clip spring 76 (FIG. 6) against the raised portion 80 of the firing bar 62 for proper engagement and for possible interaction with an end effector firing lockout mechanism (not shown). Also, this position allows for the end effector 14 to be pinched shut to facilitate insertion through a surgical entry point such as a cannula of a trocar (not shown). With reference to FIGS. 8-10, the firing bar guide 124 laterally contacts a portion of the firing bar 62 to close the corresponding portion of the lateral fluid groove 120. In FIG. 11, the EAP syringe 100 in the cylindrical cavity 90 has its distal dispensing cone 104 communicating with a radial fluid passage 220 formed in the frame 50 that communicates in turn with the lateral fluid groove 120. In FIG. 12, before installation in the surgical stapling and severing instrument 10, the EAP syringe 100 may be advantageously sealed with a disposable cap 230. In FIGS. 13-14, the EAP syringe 100 is shown without the disposable cap 230 and urged by spring 102 distally to engage the distal dispensing cone 104 into communication with the radial fluid passage 220.

It should be appreciated that one or more sensor in the surgical stapling and severing instrument 10 may sense a firing condition (e.g., movement of firing bar or mechanism coupled to the firing bar, position of the firing trigger, a separate user control to dispense, etc.) and activate dispensing control circuitry to effect dispensing.

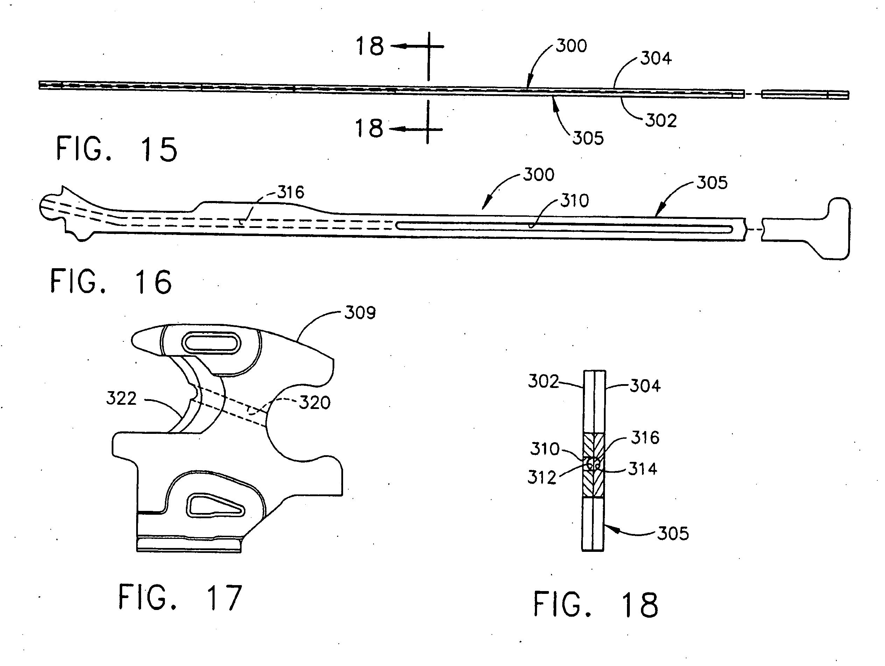

In FIGS. 15-18, an alternate two-piece firing bar 300 is formed from longitudinally laminated left half and right half firing bar portions 302, 304 that form a firing bar 305 and attached to an E-beam 309. Thereby, fluid transfer down the firing bar 300 may be further constrained. In particular, a left side fluid groove 310 in the left half firing bar portion 302 transitions distally to a pair of aligned internal fluid grooves 312, 314 respectively in the left and right half firing bar portions 302, 304, defining an internal fluid passage 316. Since the E-beam 309 is laterally thicker and of short longitudinal length, a drilled fluid passage 320 is formed therein between a cutting surface 322 and an aft edge aligned to communicate with the internal fluid passage 316.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art.

For example, while a non-articulating shaft is described herein for clarity, it should be appreciated that medical substance dispensing may be incorporated into an articulating shaft. In addition, fluid conduits may be incorporated that pass through an articulation joint of a shaft to fluid bladder actuators that close an end effector.

As another example, while both medical substance dispensing and fluid actuated anvil closing are illustrated herein, applications consistent with aspects of the invention may include either of these features. Further, for applications in which an adhesive and/or cauterizing medical substance is dispensed, it should be appreciated that features such as staples may be omitted.

As another example, while a staple applying assembly 20 is illustrated herein, it should be appreciated that other end effectors (graspers, cutting devices, etc.) may benefit from either or both of fluid controlled closing and medical substance dispensing.

As yet another example, a receptacle for the EAP syringe may be formed in the handle rather than in the elongate shaft.

As yet an additional example, a symmetric arrangement for a second EAP syringe may be formed in the elongate channel so that two medical substances may be simultaneously dispensed during firing.

As yet a further example, while a staple applying apparatus provides an illustrative embodiment, it should be appreciated that other endoscopic instruments may benefit from the ability to dispense a liquid at or near a distal end thereof. Examples of instruments that may benefit include, but are not limited to, an ablation device, a grasper, a cauterizing tool, an anastomotic ring introduction device, a surgical stapler, a linear stapler, etc. As such, those instruments that do not employ a firing bar that serves herein as a convenient fluid passage to a cutting surface may instead incorporate ducting or fluid conduits to an appropriate location.

While an electroactive polymer plunger has various advantages, it should be appreciated that other types of electrically actuated devices may be employed to dispense a medical substance through the elongate shaft to the end effector.

What is claimed is:

## Claims

1. A surgical stapling and severing instrument (10), comprising:
a handle (22) operably configured to produce a firing motion;
an elongate shaft (18) attached to the handle;
an end effector (14) distally attached to the elongate shaft and comprised of opposing jaws (12, 30) for clamping tissue;
a firing member (60) comprising a firing bar (62) received for reciprocating longitudinal motion in the elongate shaft to transfer the firing motion, a cutting edge (122) distally attached to the firing bar to sever the clamped tissue in the end effector, and a fluid passage (120) defined longitudinally in the firing bar to the cutting edge;
a staple cartridge (42) received in one of the opposing jaws responsive to the firing member to drive staples and form linear stapling lines through the clamped tissue;
control circuitry operably configured to respond to a firing condition related to distal movement of the firing member to generate a dispensing signal; and
an electrical fluid dispenser in communication with the fluid passage and responsive to the dispensing signal to dispense a medical substance along the fluid passage to the cutting surface,
wherein the electrical fluid dispenser comprises a syringe (100) disposed within the elongate shaft and comprising an internal volume (90) adapted to be filled with a medical substance (112), a dispensing opening (104) communicating with the fluid passage, and a plunger operatively configured to extend into the internal volume to dispense the medical substance through the dispensing opening when electrically activated by the dispensing signal from the control circuitry.

2. The surgical instrument of claim 1, wherein the electrical fluid dispenser further comprises an electroactive polymer actuator.

3. The surgical instrument of claim 2, wherein the plunger comprises the electroactive actuator, and wherein the electroactive actuator is operatively configured to extend the plunger into a portion of the syringe filled with the medical substance.

4. The surgical instrument of claim 3, further comprising an exteriorly accessible receptacle operatively configured to receive a filled syringe and to extract a spent syringe.

5. The surgical instrument of claim 1, wherein the fluid passage comprises a lateral groove in the firing bar.

6. The surgical instrument of claim 1, wherein the firing bar (300) comprises a longitudinal laminate of a left half and a right half (302, 304) further comprising an internal fluid passage (316) defined there between.

7. The surgical instrument of claim 1, wherein the syringe contains the medical substance selected from a group consisting of an anesthetic, an adhesive, a cauterizing substance, an antibiotic substance, and a coagulant.

8. The surgical instrument of claim 1, wherein the plunger is an electroactive polymer plunger operatively configured to expand when activated into the internal volume to dispense the medical substance through the dispensing opening; and wherein the control circuitry is operably configured to generate a dispensing signal to the electoractive polymer plunger to command dispensing.

## Patentansprüche

1. Chirurgisches Heft- und Schneidinstrument (10), das aufweist:
einen Griff (22), der betrieblich so ausgelegt ist, dass er eine Auslösebewegung erzeugt;
einen länglichen Schaft (18), der an dem Griff angebracht ist;
einen End-Effektor (14), der distal an dem länglichen Schaft angebracht ist und aus gegenüberstehenden Backen (12, 30) zum Einklemmen von Gewebe besteht;
ein Auslöseelement (60), das eine Auslösestange (62), welche für die Hin- und Herbewegung in Längsrichtung in dem länglichen Schaft aufgenommen ist, um die Auslösebewegung zu übertragen, eine Schneidkante (122), die distal an der Auslösestange angebracht ist, um das eingeklemmte Gewebe in dem End-Effektor zu schneiden, und einen Fluiddurchlass (120), der in Längsrichtung in der Auslösestange zu der Schneidkante definiert ist, aufweist;
ein Klammermagazin (42), das in einer der gegenüberstehenden Backen aufgenommen ist, welches ansprechend auf das Auslöseelement Klammern treibt und lineare Klammerlinien durch das eingeklemmte Gewebe bildet;
Steuerschaltung, die betrieblich so ausgelegt ist, dass sie auf einen Auslösezustand in Bezug auf die distale Bewegung des Auslöseelementes antwortet, um ein Abgabesignal zu erzeugen; und
eine elektrische Abgabeeinheit für Fluid, die in Verbindung mit dem Fluiddurchlass steht und auf das Abgabesignal anspricht, um eine medizinische Substanz entlang dem Fluiddurchlass zu der Schneidfläche abzugeben,
wobei die elektrische Abgabeeinheit für Fluid eine Spritze (100) aufweist, die innerhalb des länglichen Schaftes angeordnet ist und ein inneres Volumen (90), das dazu ausgelegt ist, mit einer medizinischen Substanz (112) gefällt zu werden, eine Abgabeöffnung (104), die mit dem Fluiddurchlass in Verbindung ist, und einen Stempel, der betrieblich so ausgelegt ist, dass er sich in das innere Volumen erstreckt, um die medizinische Substanz durch die Abgabeöffnung abzugeben, wenn er durch das Abgabesignal von der Steuerschaltung elektrisch aktiviert wird, aufweist.

2. Chirurgisches Instrument nach Anspruch 1, bei dem die elektrische Abgabeeinheit für Fluid weiter ein Betätigungselement aus elektroaktivem Polymer aufweist.

3. Chirurgisches Instrument nach Anspruch 2, bei dem der Stempel das elektroaktive Betätigungselement aufweist und bei dem das elektroaktive Betätigungselement betrieblich so ausgelegt ist, dass es den Stempel in einen Bereich der Spritze fährt, der mit der medizinischen Substanz gefüllt ist.

4. Chirurgisches Instrument nach Anspruch 3, das weiterhin eine von außen zugängliche Aufnahme aufweist, die betrieblich so ausgelegt ist, dass sie eine gefüllte Spritze aufnimmt und eine gebrauchte Spritze austrägt.

5. Chirurgisches Instrument nach Anspruch 1, bei dem der Fluiddurchlass eine seitliche Nut in der Auslösestange umfasst.

6. Chirurgisches Instrument nach Anspruch 1, bei dem die Auslösestange (300) ein in Längsrichtung verlaufendes Laminat aus einer linken Hälfte und einer rechten Hälfte (302, 304) aufweist, das weiterhin einen dazwischen definierten internen Fluiddurchlass (316) aufweist.

7. Chirurgisches Instrument nach Anspruch 1, bei dem die Spritze die medizinische Substanz enthält, die aus einer Gruppe bestehend aus einem Anästhetikum, einem Klebmittel, einer kauterisierenden Substanz, einer antibiotischen Substanz und einem Gerinnungsmittel ausgewählt ist.

8. Chirurgisches Instrument nach Anspruch 1, bei dem der Stempel ein Stempel mit elektroaktivem Polymer ist, der betrieblich so ausgelegt ist, dass er sich, wenn er aktiviert wird, in das innere Volumen aufweitet, um die medizinische Substanz durch die Abgabeöffnung abzugeben; und bei dem die Steuerschaltung betrieblich so ausgelegt ist, dass sie ein Abgabesignal für den Stempel mit elektroaktivem Polymer erzeugt, um das Abgeben zu befehlen.

## Revendications

1. Instrument chirurgical d'agrafage et de découpe (10), comprenant :
✔ une poignée (22) configurée de manière opérationnelle pour produire un mouvement de déclenchement ;
✔ un arbre allongé (18) fixé à la poignée ;
✔ un effecteur d'extrémité (14) fixé de manière distale à l'arbre allongé et comprenant des mâchoires opposées (12, 30) pour fixer le tissu ;
✔ un élément de déclenchement (60) comprenant une barre de déclenchement (62) reçue de façon à permettre le mouvement longitudinal en va-et-vient dans l'arbre allongé, afin de transférer le mouvement de déclenchement, un bord de coupe (122) fixé de manière distale à la barre de déclenchement pour découper le tissu fixé dans l'effecteur d'extrémité, et un passage de fluide (120) défini longitudinalement dans la barre de déclenchement au bord de coupe ;
✔ une cartouche d'agrafes (42), reçue dans l'une des mâchoires opposées en réponse à l'élément de déclenchement, pour entraîner les agrafes et former des lignes d'agrafage linéaires à travers le tissu fixé ;
✔ un circuit de commande configuré de manière opérationnelle pour répondre à une condition de déclenchement liée à un mouvement distal de l'élément de déclenchement, afin de générer un signal de distribution ; et
✔ un distributeur de fluide électrique en communication avec le passage de fluide et sensible au signal de distribution, afin de distribuer une substance médicale le long du passage de fluide vers la surface de coupe,
dans lequel le distributeur de fluide électrique comprend une seringue (100), disposée dans l'arbre allongé et comprenant un volume interne (90) adapté pour être rempli d'une substance médicale (112), une ouverture de distribution (104) en communication avec le passage de fluide et un piston configuré de manière opérationnelle pour s'étendre dans le volume interne, afin de distribuer la substance médicale à travers l'ouverture de distribution quand elle est électriquement activée par le signal de distribution depuis le circuit de commande.

2. Instrument chirurgical selon la revendication 1, dans lequel le distributeur de fluide électrique comprend en outre un actionneur polymère électro-actif.

3. Instrument chirurgical selon la revendication 2, dans lequel le piston comprend l'actionneur électro-actif et dans lequel l'actionneur électro-actif est opérationnellement configuré pour étendre le piston dans une partie de la seringue remplie de la substance médicale.

4. Instrument chirurgical selon la revendication 3, comprenant en outre un réceptacle accessible extérieurement, configuré de manière opérationnelle pour recevoir une seringue remplie et pour extraire une seringue vide.

5. Instrument chirurgical selon la revendication 1, dans lequel le passage de fluide comprend une cannelure latérale dans la barre de déclenchement.

6. Instrument chirurgical selon la revendication 1, dans lequel la barre de déclenchement (300) comprend un laminé longitudinal d'une moitié gauche et d'une moitié droite (302, 304) comprenant en outre un passage de fluide interne (316) défini entre eux.

7. Instrument chirurgical selon la revendication 1, dans lequel la seringue contient la substance médicale choisie dans un groupe constitué d'un anesthésique, d'un adhésif, d'une substance de cautérisation, d'une substance antibiotique, et d'un coagulant.

8. Instrument chirurgical selon la revendication 1, dans lequel le piston est un piston polymère électro-actif, opérationnellement configuré pour se dilater lorsqu'il est activé dans le volume interne, pour distribuer la substance médicale à travers l'ouverture de distribution, et dans lequel le circuit de commande est opérationnellement configuré pour générer un signal de distribution au piston polymère électro-actif, afin de commander la distribution.
